# EUROPÄISCHE PATENTANMELDUNG

(11) **EP 2 116 180 A1**
(43) Veröffentlichungstag der Anmeldung: **11.11.2009**
(21) Anmeldenummer: 08155742.3
(22) Anmeldetag: 06.05.2008
(51) Int. Cl.: A61B 5/00, B01L 3/00, G01N 21/86, G01N 33/487

(54) **Diagnostische Bandeinheit und diagnostisches Messsystem**

(71) Anmelder: F.HOFFMANN-LA ROCHE AG, 4070 Basel (CH); Roche Diagnostics GmbH, 68305 Mannheim (DE)
(72) Erfinder: Seelig, Peter, 60318 Frankfurt (Main) (DE); Leininger, Helmut, 68259 Mannheim (DE)
(74) Vertreter: Pfiz, Thomas

(57) **Zusammenfassung**

Die Erfindung betrifft eine diagnostische Bandeinheit mit einem auf einer Spule aufwickelbaren Testband (12), das aus einem Transportband (18) und einer Vielzahl von darauf aufgebrachten Testelementen (20) besteht, wobei die Testelemente (20) eine analytische Reagenzschicht (34), eine die Reagenzschicht (34) tragende Trägerfolie (36) und ein die Trägerfolie (36) mit dem Transportband (18) verbindendes Klebebandstück (28) aufweisen, und wobei die Reagenzschicht (34) an ihrer von der Trägerfolie (36) abgewandten Vorderseite (24) zum Aufbringen einer Probensubstanz ausgebildet ist. Erfindungsgemäß wird vorgeschlagen, dass die Testelemente (20) im Verbund mit dem lichtdurchlässigen Transportband (18) jeweils ein optisches Mehrschichtsystem für eine rückseitige reflexionsphotometrische Vermessung der Reagenzschicht (34) bilden.

## Beschreibung

Die Erfindung betrifft eine Diagnostische Bandeinheit, insbesondere Bandkassette für Blutzuckertests, mit einem als Bandwickel auf einer Spule aufgewickelten oder aufwickelbaren Testband, das aus einem Transportband und einer Vielzahl von darauf aufgebrachten Testelementen besteht, wobei die Testelemente eine analytische Reagenzschicht, eine die Reagenzschicht tragende Trägerfolie und ein die Trägerfolie mit dem Transportband verbindendes Klebebandstück aufweisen, und wobei die Reagenzschicht an ihrer von der Trägerfolie abgewandten Vorderseite zum Aufbringen einer Probensubstanz vorgesehen ist. Die Erfindung betrifft weiter ein Messsystem zum Einsatz einer solchen Bandeinheit.

Solche Bandeinheiten wurden vor allem für Blutzuckertests konzipiert, um gegenüber den am Markt befindlichen Teststreifensystemen die Benutzerfreundlichkeit weiter zu verbessern. So kann im Sinne einer vereinfachten Handhabung auf einem aufrollbaren Transportband eine hohe Anzahl von Testelementen kompakt bevorratet und durch den Bandtransport nach Gebrauch auch wieder entsorgt werden. Zweckmäßig ist eine solche Bandeinheit in Form einer Kassette als Verbrauchsmaterial in ein Handgerät einsetzbar, um dem Benutzer weitgehend automatisch ablaufende Selbsttests zu ermöglichen.

Bei den herkömmlichen trockenchemischen Teststreifen werden Reagenzfelder auf einen vergleichsweise dicken Reagenzträger aufgebracht. Bei einem solchen Einschicht-System gestaltet sich eine reflexionsoptische Vermessung relativ problemlos. Ein in Form eines Bandwickels aufgespultes Testband mit einer Vielzahl von Tests lässt sich damit jedoch nicht realisieren.

Ausgehend hiervon liegt der Erfindung die Aufgabe zugrunde, die im Stand der Technik bekannten Bandkonzepte weiter zu entwickeln und eine auch als Massenprodukt für eine zuverlässige Messung konzipierte Bandeinheit der eingangs angegebenen Art sowie ein Messsystem dafür anzugeben.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen vorderseitigen Probenauftrag und eine rückseitige Vermessung bei einem Testband mit voneinander beabstandeten Testfeldern zu ermöglichen. Dementsprechend wird erfindungsgemäß vorgeschlagen, dass die Testelemente im Verbund mit dem lichtdurchlässigen Transportband jeweils ein optisches Mehrschichtsystem für eine rückseitige reflexionsphotometrische Vermessung der Reagenzschicht bilden. Durch den mehrschichtigen Aufbau ist es möglich, relativ dünne vorgefertigte Testträger in einen Bandwickel zu integrieren, wobei durch den einheitlichen optischen Mehrschichtverbund eine messtechnische Erfassung der Remission an einem freiliegenden Testbandabschnitt unabhängig von der Handhabungsseite erfolgen kann.

Um ein Messsignal in der für diagnostische Anwendungen geforderten Güte zu erhalten, ist es von besonderem Vorteil, wenn der Brechungsindex und/oder die Transmission und/oder die Trübung der durch die Trägerfolie, das Klebebandstück und das Transportband gebildeten Schichten des Mehrschichtsystems innerhalb vorgegebener Toleranzen aufeinander abgestimmt sind. Besonders günstig ist es in diesem Zusammenhang, wenn der Brechungsindex des Transportbands, des Trägerbands und des Klebebandstücks jeweils zwischen 1,4 und 1,7, vorzugsweise 1,5 bis 1,6 beträgt.

Eine weitere Verringerung von Störeinflüssen lässt sich dadurch erreichen, dass die einzelnen Schichten des Mehrschichtsystems eine Brechungsindexdifferenz von maximal 0,2 und vorzugsweise weniger als 0,1 aufweisen.

Hinsichtlich einer Optimierung der durch das Mehrschichtsystem insgesamt realisierbaren Parameter ist es vorteilhaft, wenn der Gesamtbrechungsindex etwa 1,5 beträgt. Um Reflexionseffekte in einem unkritischen Bereich zu halten, sollte die Abweichung im Brechungsindex möglichst kleiner 0,1 betragen.

Weitere Verbesserungen lassen sich dadurch erzielen, dass das Transportband, das Klebebandstück und die Trägerfolie im sichtbaren Wellenlängenbereich eine Transmission von jeweils mehr als 80%, vorzugsweise 85% bis 92% besitzen und dass die Gesamttransmission des Mehrschichtsystems im sichtbaren Wellenlängenbereich mindestens 80% beträgt.

Für eine reproduzierbare Messung ist es auch von besonderer Bedeutung, dass die Transmissionstoleranz für die Gesamtheit der Testelemente eines Testbands unter 5% liegt.

Um Streuverluste hinreichend zu reduzieren, ist es vorteilhaft, wenn die optische Trübung des Trägerbands und des Klebebandstücks im sichtbaren Wellenlängenbereich weniger als 10%, insbesondere etwa 8% beträgt. Günstig ist es auch, wenn die optische Trübung des Transportbands im sichtbaren Wellenlängenbereich weniger als 3%, insbesondere etwa 2,5% beträgt, und wenn die gesamte optische Trübung des Mehrschichtsystems im sichtbaren Wellenlängenbereich weniger als 20%, vorzugsweise etwa 15% beträgt.

Um herstellungsbedingte Störeinflüsse berücksichtigen zu können, sollte eine Trübungsabnahme des Mehrschichtsystems in einem gegebenen Zeitintervall insbesondere von etwa 1 bis 2 Wochen nach dessen Herstellung erfolgen, wobei anschließend die Trübung vergleichsweise konstant bleiben sollte.

Um Varianzen innerhalb einer gegebenen Bandeinheit hinreichend zu begrenzen, sollte die Trübungstoleranz für die Gesamtheit der Mehrschichtsysteme eines Testbands weniger als 5% betragen.

Vorteilhafterweise wird ein mehrschichtiges Klebebandstück eingesetzt, das aus einem beidseitig mit einer Klebemittelschicht versehenen transparenten Foliensubstrat besteht.

Für den vorgesehenen Einsatzzweck ist es auch günstig, wenn das Transportband und die Trägerfolie aus einem PET-Film bestehen. Allgemein sind Polymerfolien vorteilhaft einsetzbar. Beispielhaft sind als vorteilhafte Materialien neben Polyethylenterephtalat (PET) auch Polyvinylfluorid (PVF), Polyethylen (PE), Polypropylen (PP), Polyvinylidendifluorid (PVDF), Polyvinylchlorid (PVC), Polystyrol (PS), Ethen/Tetrafluorethylen-Copolymere (ETFE), Polycarbonat und Propylencarbonat zu nennen.

Für eine praxisgerechte Realisierung ist es auch von Vorteil, wenn die Dicke der Trägerfolie zwischen 20 und 25µm, die Dicke des Transportbands zwischen 10 und 15µm und die Dicke des Klebebandstücks zwischen 30 und 50µm beträgt.

Zur Berücksichtigung von Störeinflüssen, die sich durch einen spezifischen Aufbau ergeben, ist es vorteilhaft, wenn ein in der vorzugsweise durch eine Körperflüssigkeit, insbesondere Blut gebildeten Probensubstanz enthaltener Analyt durch eine relative Remissionsmessung bestimmbar ist, wobei den Testelementen Kalibrierdaten zugeordnet sind, welche die Konzentration des Analyten in Abhängigkeit von der gemessenen Remission definieren.

Ein weiterer Aspekt der Erfindung betrifft ein diagnostisches Messsystem, insbesondere für Blutzuckertests, mit einer erfindungsgemäßen diagnostischen Bandeinheit und einer auf die Rückseite der Reagenzschicht des in einer Messposition befindlichen Testelements ausgerichteten reflexionsphotometrischen Anordnung, die eine Lichtquelle und einen Photodetektor umfasst, wobei der Detektor außerhalb des direkten Reflexionspfades von der Lichtquelle durch das Mehrschichtsystem hindurch auf die Reagenzschicht eingestrahlten Messlichts angeordnet ist.

In diesem Zusammenhang ist es für eine Beleuchtungsoptimierung von Vorteil, wenn die Lichtquelle einen Leuchtfleck von weniger als 1 mm² auf der Rückseite der Reagenzschicht erzeugt, wobei eine Körnung der Reagenzschicht als Streukörper dient.

Im Folgenden wird die Erfindung anhand des in der Zeichnung schematisch dargestellten Ausführungsbeispiels näher erläutert. Es zeigen:
- Fig. 1: eine diagnostische Bandeinheit in Form einer Bandkassette in geschnittener perspektivischer Darstellung;
- Fig. 2: das Testband der Bandkassette in einem ausschnittsweisen Längsschnitt im Bereich eines Testelements; und
- Fig. 3: eine auf das Testband ausgerichtete reflektometrische Messanordnung in schaubildlicher Darstellung.

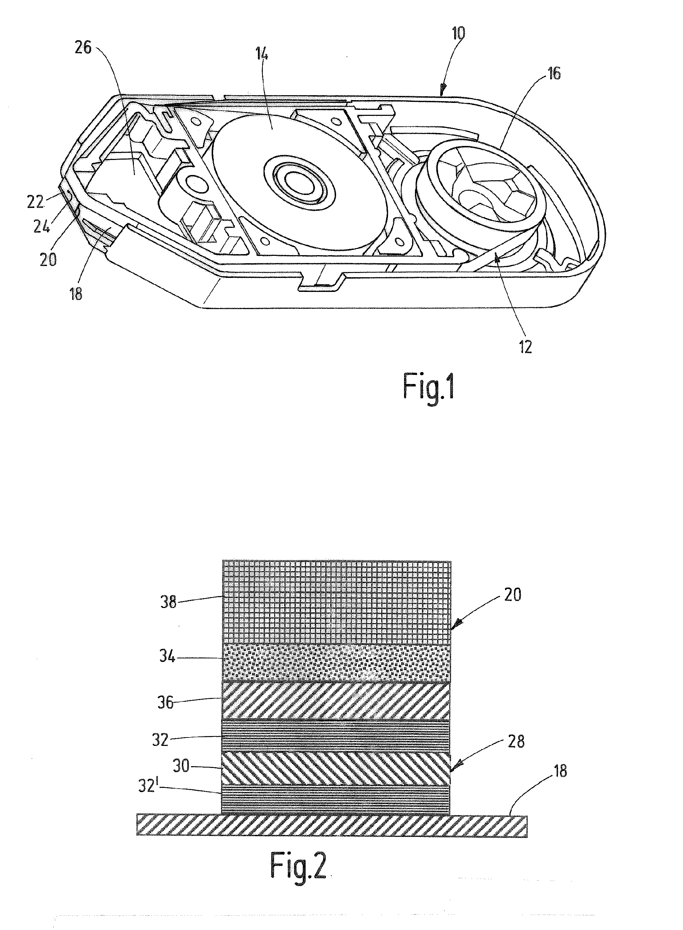

Die in Fig. 1 dargestellte Bandkassette 10 lässt sich als Verbrauchseinheit in ein nicht gezeigtes Handgerät zur Durchführung einer Vielzahl von Blutzucker-Selbsttests einsetzen. Die Kassette umfasst ein Testband 12, eine Abwickelspule 14 zum Abwickeln von ungebrauchtem Testband und eine Aufwickelspule 16 zum Aufwickeln von gebrauchtem Testband, wobei das Testband 12 ein aufrollbares Transportband 18 und eine Vielzahl von darauf im Abstand voneinander aufgebrachten Testelementen 20 aufweist.

Das unbenutzte Testband wird in Form eines Bandwickels auf der Abwickelspule 14 vor Umwelteinflüssen geschützt aufbewahrt. Das Testband 12 lässt sich über einen an der Aufwickelspule 16 angreifenden Drehantrieb vorspulen, so dass die Testelemente 20 an einer Applikationsstelle 22 für den Benutzer sukzessive bereitstellbar sind. Dort lässt sich an der freiliegenden Vorderseite 24 des jeweils aktiven Testelements 20 auf einfache Weise ein Blutstropfen auftragen, während durch eine in die Messkammer 26 der Kassette 10 eingreifende geräteseitige Messanordnung eine rückseitige reflexionsphotometrische Vermessung erfolgt. Der verbrauchte Testbandabschnitt wird auf der Aufwickelspule 16 entsorgt. Auf diese Weise können ca. 50 Tests verarbeitet werden, ohne dass ein Geräteeingriff oder umständliche Bedienschritte durch den Benutzer erforderlich wären.

Wie in Fig. 2 veranschaulicht, bildet das Transportband 18 mit den aufgebrachten Testelementen 20 einen mehrlagigen Verbundaufbau bzw. ein Mehrschichtsystem, dem ein vereinfachtes Herstellungsverfahren zugrunde liegt, wie es in der EP-A 1 593 434 beschrieben ist, auf die in diesem Zusammenhang Bezug genommen wird. Die Testelemente 20 werden dabei gleichsam als Testetiketten über ein doppelseitiges Klebebandstück 28 auf das Transportband 18 aufgeklebt. Das Klebebandstück 28 umfasst zu diesem Zweck eine Liner-Folie 30, die beidseitig mit einer Klebeschicht 32, 32' versehen ist. Die Nachweisreaktion erfolgt in einer dünnen Reagenzschicht 34, die auf eine Trägerfolie 36 als Trockensubstanz aufgebracht ist und darüber auf dem Klebebandstück 28 gehalten wird. Die Stärke einer Farbänderung des Reagenz steht dabei in einem funktionellen Zusammenhang mit der Konzentration des zu messenden Analyten (hier Blutglukose). An der Applikationsseite der Reagenzschicht 34 kann durch eine netzförmige Spreitschicht 38 eine flächige Verteilung der aufgetragenen Körperflüssigkeit erreicht werden.

Um eine zuverlässige Remissionsmessung zu ermöglichen, sind in dem vorstehend beschriebenen mehrschichtigen Bandaufbau besondere Vorkehrungen und Abstimmungen hinsichtlich der optischen Eigenschaften zu treffen, wie sie in Tabelle I zusammengefasst sind.

Das lange flexible Transportband 18 besteht vorteilhafterweise aus PET und besitzt eine Dicke d von etwa 12µm. Der Brechungsindex n beträgt 1,6. Es ist weitgehend lichtdurchlässig bzw. transparent, wobei die spektrale Transmission T im Wellenlängenbereich zwischen 400 und 900nm mehr als 85% betragen sollte. Daneben beeinflussen auch die Oberflächen- und Volumenstreuung die Signalqualität. Beides zusammen kann durch eine Trübungsmessung erfasst werden. Zweckmäßig sollte die Trübung (Haze H) bei etwa 2,5% liegen. Die genannten Parameter wurden mit einem standardisierten Messverfahren gemäß der Norm ASTM-D 1003-61 Methode A bestimmt (Standard Test Method for Haze and Luminous Transmittance of Transparent Plastics). Von besonderer Wichtigkeit ist auch die Einhaltung von Toleranzbereichen (Toleranzbreiten), um in einer Massenproduktion die Messpräzision innerhalb der zugelassenen Grenzen zu gewährleisten. Für das in der benötigten Länge abgeschnittene Transportband 18 sollte die Transmissionstoleranz ΔT weniger als 2% betragen, während die Trübung weniger als 0,5% variieren sollte.

Auch die übrigen Schichten in dem Mehrschichtsystem sind gemäß der Tabelle I speziell aufeinander abgestimmt.

**Tabelle I:**

| | d | n | T | ΔT | H | ΔH |
|---|---|---|---|---|---|---|
| Transportband 18 | 12 µm | 1,6 | >88% | <2% | 2,5% | <0,5% |
| Klebebandstück 28 | 42 µm | 1,5 | >85% | <3% | 8,0% | <3% |
| PET Trägerfolie 36 | 23 µm | 1,6 | >88% | <2% | 8,0% | <1% |
| Gesamtsystem 18,28,36 | 77 µm | 1,5 | >80% | <5% | 15% | <5% |

In der letzten Zeile der Tabelle sind die einheitlich ermittelten Messwerte für den gesamten Aufbau aus Transportband 18, Klebebandstück 28 und Trägerfolie 36 angegeben. Für das Gesamtsystem wurde eine besondere Problematik hinsichtlich der zeitlichen Konstanz der Messwerte dahingehend beobachtet, dass innerhalb der ersten 1 bis 2 Wochen nach der Herstellung eine Abnahme der Trübungswerte stattfand. Danach bleiben die Messwerte im Wesentlichen zeitlich stabil. Die Einzelkomponenten zeigen vor ihrem Zusammenfügen ein solches Verhalten nicht. Dies lässt sich dadurch erklären, dass die anfangs erhöhte Trübung auf den Einschluss von Luftbläschen beim Zusammenfügen der Einzelkomponenten zu dem Gesamtsystem und daraus resultierender erhöhter Streuung verursacht wird. Der zeitliche Abfall der gemessenen Trübungswerte wird dann durch das Entweichen der eingeschlossenen Luft durch Diffusion verursacht.

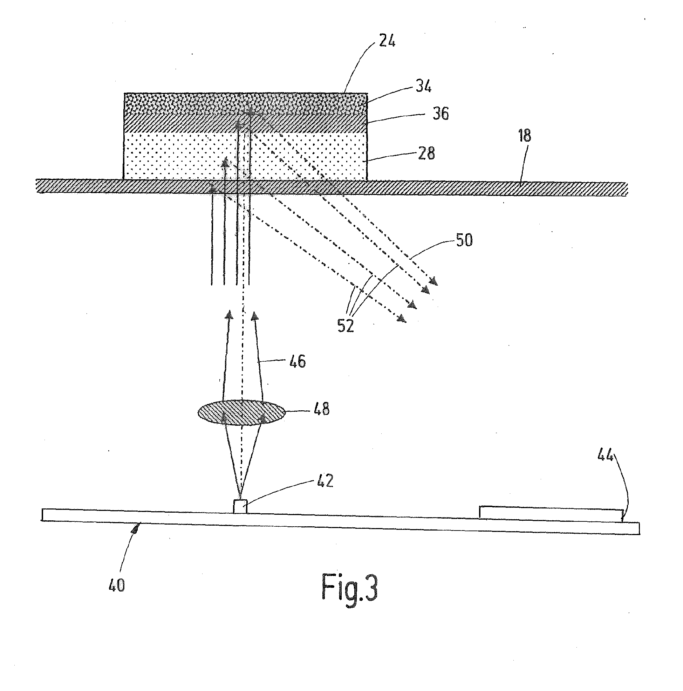

Fig. 3 veranschaulicht ein diagnostisches Messsystem, wie es in einem tragbaren Blutzuckermessgerät bei eingelegter Bandkassette 10 realisiert ist. Die rückseitige Vermessung der Reagenzschicht 34 erfolgt durch eine geräteseitige reflexionsphotometrische Anordnung 40, die eine Lichtquelle (LED 42) und einen optischen Detektor (Photodiode 44) umfasst. Das eingestrahlte Lichtbündel 46 wird über eine Optik (Sammellinse 48) auf einen kleinen Leuchtfleck auf der Rückseite der Reagenzschicht 44 fokussiert, wobei deren Körnung als Streukörper dient. Der Detektor 44 liegt dabei außerhalb des Ausfallwinkelbereichs des direkt reflektierten Lichtanteils, so dass im Wesentlichen nur das Streulicht erfasst wird.

Das System ist somit derart ausgelegt, dass das durch die Reagenzschicht 34 gebildete Testfeld mit hoher Intensität beleuchtet wird. Dabei tritt das Messlicht mit dem Reagenz in Wechselwirkung und wird in Abhängigkeit von Absorption und Transmission gestreut. Das gestreute Nutzlicht 50 fällt entsprechend dem Nachweisraumwinkel auf den Detektor 44. Dort wird allerdings auch das an den übrigen Komponenten 18, 28, 36 gestreute bzw. reflektierte Störlicht 52 nachgewiesen (der Strahlengang ist in Fig. 3 symbolisch vereinfacht). Die Güte des Messsignals ergibt sich daher aus dem Verhältnis von Nutz- zu Störlicht. Durch die beschriebene Anpassung der optischen Eigenschaften der im Strahlengang befindlichen Elemente bezüglich Brechungsindex, Transmission, Absorption und Streuvermögen im Bereich der Beleuchtungswellenlänge wird erreicht, dass das Messsignal der geforderten Güte entspricht.

Der funktionelle Zusammenhang zwischen der gemessenen Remission und der Konzentration des Analyten lässt sich durch eine Kalibrierkurve beschreiben. Das über eine Mikroelektronik gesteuerte Messgerät kann so zu jeder ermittelten Remission den korrekten Konzentrationswert zuordnen und über ein Display anzeigen. Die Bestimmung der Kalibrierkurve erfolgt an den gleichen Reagenzien in der beschriebenen Testfeldanordnung. Die sich durch den spezifischen Aufbau ergebenden Störlichtanteile können daher bei der Kalibrierung berücksichtigt werden. Die prozessbedingten Schwankungen der unterschiedlichen Störeinflüsse treten innerhalb einer gewissen Toleranz auf. Die zulässige Toleranz für diese Störeinflüsse ergibt sich aus den vorgegebenen bzw. erlaubten Grenzen auf Konzentrationsebene des betreffenden Analyten.

## Patentansprüche

1. Diagnostische Bandeinheit, insbesondere Bandkassette (10) für Blutzuckertests, mit einem als Bandwickel auf einer Spule aufgewickelten oder aufwickelbaren Testband (12), das aus einem Transportband (18) und einer Vielzahl von darauf aufgebrachten Testelementen (20) besteht, wobei die Testelemente (20) eine analytische Reagenzschicht (34), eine die Reagenzschicht (34) tragende Trägerfolie (36) und ein die Trägerfolie (36) mit dem Transportband (18) verbindendes Klebebandstück (28) aufweisen, und wobei die Reagenzschicht (34) an ihrer von der Trägerfolie (36) abgewandten Vorderseite (24) zum Aufbringen einer Probensubstanz ausgebildet ist, **dadurch gekennzeichnet, dass** die Testelemente (20) im Verbund mit dem lichtdurchlässigen Transportband (18) jeweils ein optisches Mehrschichtsystem für eine rückseitige reflexionsphotometrische Vermessung der Reagenzschicht (34) bilden.

2. Diagnostische Bandeinheit nach Anspruch 1, **dadurch gekennzeichnet, dass** der Brechungsindex und/oder die Transmission und/oder die Trübung der durch die Trägerfolie (36), das Klebebandstück (28) und das Transportband (18) gebildeten Schichten des Mehrschichtsystems innerhalb vorgegebener Toleranzen aufeinander abgestimmt sind.

3. Diagnostische Bandeinheit nach Anspruch 1 oder 2, **dadurch gekennzeichnet, dass** der Brechungsindex des Transportbands (18), der Trägerfolie (36) und des Klebebandstücks (28) jeweils zwischen 1,4 und 1,7, vorzugsweise 1,5 bis 1,6 beträgt.

4. Diagnostische Bandeinheit nach einem der Ansprüche 1 bis 3, **dadurch gekennzeichnet, dass** die einzelnen Schichten des Mehrschichtsystems eine Brechungsindexdifferenz von maximal 0,2, vorzugsweise weniger als 0,1 aufweisen.

5. Diagnostische Bandeinheit nach einem der Ansprüche 1 bis 4, **dadurch gekennzeichnet, dass** der Gesamtbrechungsindex des Mehrschichtsystems 1,5 beträgt.

6. Diagnostische Bandeinheit nach einem der Ansprüche 1 bis 5, **dadurch gekennzeichnet, dass** das Transportband (18), das Klebebandstück (28) und die Trägerfolie (36) im sichtbaren Wellenlängenbereich eine Transmission von jeweils mehr als 80%, vorzugsweise 85% bis 92% besitzen.

7. Diagnostische Bandeinheit nach einem der Ansprüche 1 bis 6, **dadurch gekennzeichnet, dass** die Gesamttransmission des Mehrschichtsystems im sichtbaren Wellenlängenbereich mindestens 80% beträgt.

8. Diagnostische Bandeinheit nach einem der Ansprüche 1 bis 7, **dadurch gekennzeichnet, dass** die Transmissionstoleranz für die Gesamtheit der Testelemente (20) eines Testbands (12) unter 5% liegt.

9. Diagnostische Bandeinheit nach einem der Ansprüche 1 bis 8, **dadurch gekennzeichnet, dass** die optische Trübung der Trägerfolie (36) und des Klebebandstücks (28) im sichtbaren Wellenlängenbereich weniger als 10%, insbesondere etwa 8% beträgt.

10. Diagnostische Bandeinheit nach einem der Ansprüche 1 bis 9, **dadurch gekennzeichnet, dass** die optische Trübung des Transportbands (18) im sichtbaren Wellenlängenbereich weniger als 3%, insbesondere etwa 2,5% beträgt.

11. Diagnostische Bandeinheit nach einem der Ansprüche 1 bis 10, **dadurch gekennzeichnet, dass** die gesamte optische Trübung des Mehrschichtsystems im sichtbaren Wellenlängenbereich weniger als 20%, vorzugsweise etwa 15% beträgt.

12. Diagnostische Bandeinheit nach einem der Ansprüche 1 bis 11, **dadurch gekennzeichnet, dass** die Trübung des Mehrschichtsystems in einem Zeitintervall insbesondere von etwa 1 bis 2 Wochen nach dessen Herstellung abnimmt, und dass anschließend die Trübung vergleichsweise konstant bleibt.

13. Diagnostische Bandeinheit nach einem der Ansprüche 1 bis 12, **dadurch gekennzeichnet, dass** die Trübungstoleranz für die Gesamtheit der Mehrschichtsysteme eines Testbands (12) weniger als 5% beträgt.

14. Diagnostische Bandeinheit nach einem der Ansprüche 1 bis 13, **dadurch gekennzeichnet, dass** das mehrschichtige Klebebandstück (28) aus einem beidseitig mit einer Klebemittelschicht (32,32') versehenen transparenten Foliensubstrat (30) besteht.

15. Diagnostische Bandeinheit nach einem der Ansprüche 1 bis 14, **dadurch gekennzeichnet, dass** das Transportband (18) und die Trägerfolie (36) aus einem PET-Film bestehen.

16. Diagnostische Bandeinheit nach einem der Ansprüche 1 bis 15, **dadurch gekennzeichnet, dass** ein in der vorzugsweise durch eine Körperflüssigkeit, insbesondere Blut gebildeten Probensubstanz enthaltener Analyt durch eine relative Remissionsmessung bestimmbar ist, wobei den Testelementen (20) Kalibrierdaten zugeordnet sind, welche die Konzentration des Analyten in Abhängigkeit von der gemessenen Remission definieren.

17. Diagnostisches Messsystem, insbesondere für Blutzuckertests mit einer diagnostischen Bandeinheit (10) nach einem der vorhergehenden Ansprüche und einer auf die Rückseite der Reagenzschicht (34) des in einer Messposition befindlichen Testelements (20) ausgerichteten reflexionsphotometrischen Anordnung (40), die eine Lichtquelle (42) und einen Photodetektor (44) umfasst, wobei der Photodetektor (44) außerhalb des direkten Reflexionspfades von der Lichtquelle (42) durch das Mehrschichtsystem hindurch auf die Reagenzschicht (34) eingestrahlten Messlichts angeordnet ist.

18. Diagnostisches Messsystem nach Anspruch 17, **dadurch gekennzeichnet, dass** die Lichtquelle (42) einen Leuchtfleck von weniger als 1 mm² auf der Rückseite der Reagenzschicht (34) erzeugt, wobei eine Körnung der Reagenzschicht (34) als Streukörper dient.
